# EUROPEAN PATENT SPECIFICATION

(11) **EP 4 184 067 B1**
(45) Date of publication and mention of the grant of the patent: **27.11.2024**
(21) Application number: 21843392.8
(22) Date of filing: 13.07.2021
(51) Int. Cl.: F24F 3/16, F24F 11/00, F24F 8/30, A61L 9/12, A61L 9/22, A61L 9/015, F24F 8/26, F24F 8/20, F24F 8/24, F24F 8/50, F24F 110/20, F24F 110/66, F24F 110/70, F24F 110/74, F24F 130/20

(54) **DEVICE FOR THE DISINFECTION AND PURIFICATION OF AIR AND SURFACES**
VORRICHTUNG ZUR DESINFEKTION UND REINIGUNG VON LUFT UND OBERFLÄCHEN
DISPOSITIF POUR LA DÉSINFECTION ET LA PURIFICATION D'AIR ET DE SURFACES

(30) Priority: 15.07.2020 ES 202030729
(43) Date of publication of application: 24.05.2023
(73) Proprietor: Casa Boncompte, Marc, 08224 Terrassa Barcelona (ES); Casas Boncompte, Joan Francesc, 08224 Terrassa (ES)
(72) Inventor: Casa Boncompte, Marc, 08224 Terrassa Barcelona (ES); Casas Boncompte, Joan Francesc, 08224 Terrassa (ES)
(74) Representative: Torras Toll, Jorge
(86) International application number: PCT/ES2021/070512
(87) International publication number: WO 2022/013470

(56) References cited:
- EP-A1- 1 348 448
- EP-A2- 0 529 937
- EP-A2- 0 529 937
- EP-A2- 1 466 667
- WO-A1-2015/008964
- WO-A1-2015/008964
- WO-A1-2015/121633
- WO-A1-2019/000693
- WO-A2-2012/059726
- CN-A- 110 486 866
- CN-A- 111 256 250
- CN-U- 206 929 935
- JP-A- H11 230 605
- US-A1- 2014 079 597
- US-A1- 2015 297 776
- US-A1- 2016 363 339
- US-A1- 2018 200 401
- US-A1- 2019 216 967
- US-A1- 2020 215 219

## Description

### TECHNICAL FIELD

The present invention relates to a device for disinfection and purification of air and surfaces by emission of hydroxyl radicals which improves the performance of this type of device.

### BACKGROUND OF THE INVENTION

It is a fact that people nowadays spend most of their time in enclosed spaces, both at home and in their workplaces, and are therefore immersed in an atmosphere dominated by suspended particles (pathogens) that cause respiratory ailments. However, this is not the only problem in these rooms. In recent times, we have experienced the need to keep them in safer conditions and free of pollutants. As a solution to this problem, the systematic ventilation of spaces and rooms or the use of specific devices to solve this problem is recommended.

However, room ventilation is not always possible, e.g. in airtight buildings, so means are needed to control and limit the number of airborne particles in the air.

For this function, purifying devices have been developed whose main objective is to eliminate pollutants or toxic elements by capturing the ambient air through a fan and passing it through a filter that traps harmful particles, fumes and other impurities. Along with the particles in suspension, there are other types of elements such as germs, bacteria and viruses that are more complex to eradicate and generally tend to multiply in conventional environmental control systems based on the use of filters. These devices are called passive type.

For the eradication of these pathogens in enclosed spaces, various systems have recently become widespread. There is a type called Advanced Oxidation Process (AOP) where the use of hydroxyl radicals form the basis as an airborne active disinfectant.

The oxidizing and disinfecting property of hydroxyl radicals OH° (the second most oxidizing natural product in nature after fluorine) is that they react quickly with any environmental pathogen (both in the air and on surfaces in contact with the air), absorbing hydrogen atoms from organic elements and converting them into water vapour molecules (H₂O). With this mechanism, all organic molecules are indiscriminately attacked, destroying bacterial capsules and cell walls. When the rate of cell wall damage is greater than the rate of its own repair, the cell denatures and dies. In non-biological (non-organic) organisms it reacts by producing other elements such as molecules of alcohols, carbons + O₂ and other chemical compounds.

Air purification devices by the emission of hydroxyl radicals are known which comprise a body equipped with an ionizer (ozone generator, ozone lamps or LEDs, etc.), a tank containing a reagent capable of producing hydroxyl radicals by reaction with ozone and means for causing the radical to emanate.

An example of an apparatus for generating hydroxyl radicals is described in EP0529937B1 comprising a supply of olefin to produce olefin vapour and a supply of ozone. The olefin vapour is mixed and reacts with ozone to produce hydroxyl radicals.

The disadvantage of this device is that it emits hydroxyl radicals continuously while connected, and the user must switch the equipment on and off depending on the decontamination needs of the moment.

WO2015008964 discloses a device upon which the preamble of appending claim 1 is based.

A further developed device is described in GB2485280. This device describes the incorporation of control electronics that supply power to the fan and ozone generator intermittently, so that hydroxyl radicals are released into the atmosphere intermittently or periodically. In this way, a setting of hydroxyl radicals delivered per unit time can be established.

Despite the improvement over the first device described, the second device still has the disadvantage of not being able to control the hydroxyl radicals supplied depending on the level of contamination of the air to be treated, an area that needs to be covered by the device and with the control of light periods for differentiation of day/night work cycles.

Another disadvantage of the devices described in the state of the art is that they perform their function without considering the level of ozone in the room, so that the ozone maximum levels determined by international standards can easily be exceeded and become harmful to health. Another disadvantage of the devices described in the state of the art is that it is not possible to regulate the airflow according to the current decontamination needs or the volumetric size of the area.

### OBJECT OF THE INVENTION

The object of the invention consists of a device for disinfecting and purifying air and surfaces by means of hydroxyl radical emission which improves the performance of this type of device by presenting various means of regulation and control which instantly adapt the operation of the apparatus to the degree of contamination of the air in the room, as well as to its size.

It also features improvements that allow recognition of the amount of reagent used for the reaction and emission of hydroxyl radicals, as well as a reagent cavity that optimizes the mixing of air, reagent vapour and ozone.

### EXPLANATION OF THE INVENTION AND ADVANTAGES

The novel air purification device comprises a body with a low ozone emission ionizer, with power regulation by pulse width modulation (PWM) or by voltage, a monoterpene reagent removable tank capable of producing hydroxyl radicals by reaction with ozone and air and means for causing the reagent to emanate, further comprising a central processing unit (CPU) with Wi-Fi and Bluetooth capability that receives information from a means of sensing environmental conditions, from which it manages the operation of the system to adapt the emission of hydroxyl radicals to the needs measured at the time.

These means of detection are:
- An internal air sensor that analyses volatile particulate matter, CO₂, volatile organic compounds (VOC) such as benzene, toluene, nitrobenzene, isoprene, pinene, formaldehyde, etc. which determines the air quality in the room, and other environmental aspects such as temperature or relative humidity.
- An external air sensor, which analyses and informs the device about the air quality away from the device, but within its volumetric coverage area, which communicates with the CPU via Bluetooth or Wi-Fi.
- An ozone sensor, which detects ozone levels in order not to exceed regulatory values and to ensure proper decomposition of the monoterpene.
- A luminosity sensor for automated day cycle management which regulates the activity levels of the device according to the need for action, so that during the day or when the room light is on, it means that there is human movement within the action zone and therefore more pollutants, At night or when the light is off, the equipment will automatically reduce its level of action by reducing the systems that can produce more noise, limiting the emission of hydroxyl, turning off all light and sound indicators and ultimately consuming less energy (low efficiency cycle).

Operationally, the device has a room air inlet with a filter capable of retaining suspended particles, which communicates with an Air intake duct in which the internal air sensor is located.

This Air intake duct is connected to the upper part of the removable monoterpene reagent tank and the airflow in this area can be regulated by means of a mechanical or electromechanical flow control device to increase or decrease the amount of monoterpene vapour sucked in by means of the Venturi effect. This air, together with the monoterpene vapour, is fed into a reactive chamber fitted with spiral-shaped guides, into which converges an ozone supply channel leads the ozone produced from the ionizer into the reactive chamber.

In the reactive chamber, by means of the spiral guides, the filtered air coming from the inlet channel is mixed in an optimized way with the monoterpene vapour generated by the emanation of the reagent and the ozone generated in the ionizer, also is regulated the flow rate passes through its interior, by the Air intake duct and by the ozone supply channel to facilitate the chemical reaction and generating the hydroxyl radical through the suction of a turbine with regulated by pulse width modulation (PWM) or voltage, located at the output of the reactive chamber, which adjusts the flow rate to the volume of the passenger compartment.

Another novel aspect of the invention refers to the incorporation of control means with safety protection that guarantee the proper use of the reagent, the use of an original product (understood as a product authorized by the manufacturer) and the initial volume, remaining volume, and use of the monoterpene reagent tank. These means consist of a radio frequency identification system (RFID) comprising a transponder or tag incorporated in the reagent tank consisting of an antenna, a radio transducer, and a chip, which transmits the identification information to an RFID reader integrated in the body of the device and consisting of an antenna, a transceiver and a decoder. When the reader picks up the RFID signal, it extracts the information contained on the chip and transmits it to the CPU.

The means for causing the monoterpene reagent to emanate consists of a wick inserted in the reagent tank, which can be of various sizes and protrude a certain height through the mouth of the reagent tank, which communicates with the Air intake duct. The air flow in this area can be regulated by means of a flow control device consisting of a changeable fixed part of various sizes or by an electromechanical mobile system, so that the monoterpene vapour generated is sucked by the Venturi effect through the Air intake duct and introduced into the reactive chamber.

The central processing unit is linked to a device management (control) touch display, through which the user can select different operating options and enter values that condition the operation of the device, such as room volume.

This central control unit has software that incorporates a calendar for programming the operating cycles in high efficiency, low efficiency, standby, on and off. With days of the week and times of day.

### DRAWINGS AND REFERENCES

To illustrate the above, the present descriptive report is accompanied by drawings in which an example of an embodiment is shown, which is only illustrative and not restrictive of the practical possibilities of the invention.

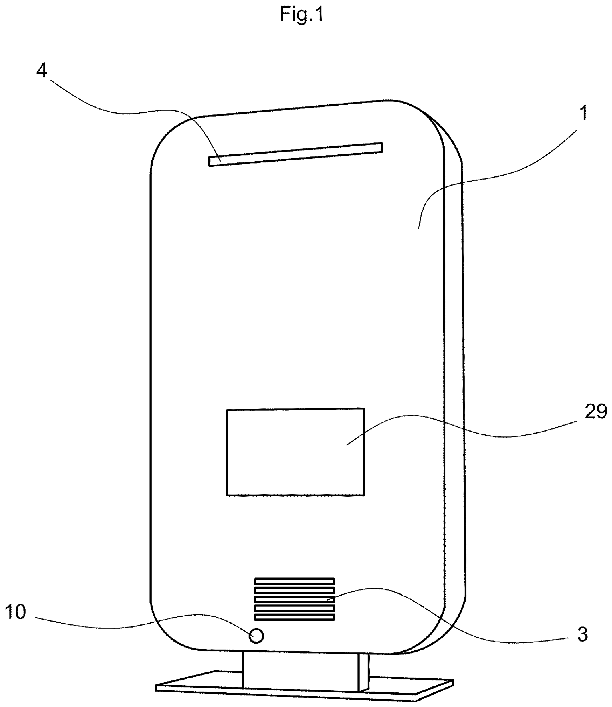

Figure 1 shows a front view of the air purification device.

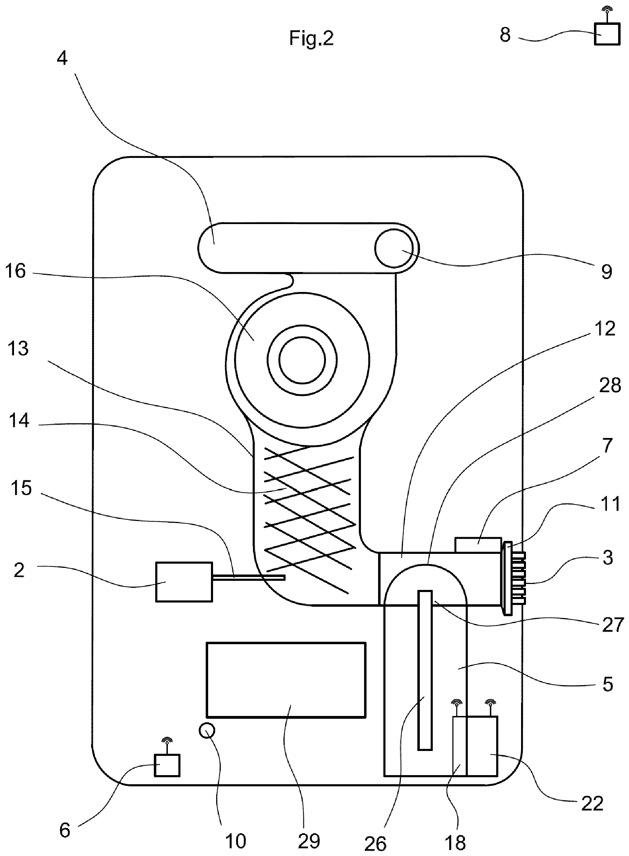

Figure 2 shows a schematic view of the interior of the device.

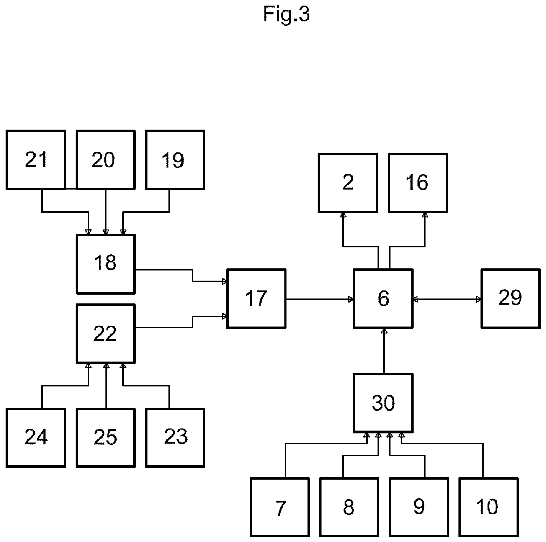

Figure 3 represents a working diagram of the invention.

The following references are indicated in these figures:
1- Body
2- Ionizer
3- Air intake
4- Output hydroxyl radicals
5- Removable tank
6- Central Processing Unit CPU
7- Internal air sensor
8- External air sensor
9- Ozone sensor
10- Luminosity sensor
11- Carbon filter
12-Air intake duct
13- reactive chamber
14- spiral-shaped guides
15- Channel ozone supply
16- Turbine
17- Radio frequency identification system
18- Transponder
19- Antenna
20- Radio transducer
21- Chip
22- RFID reader
23- Antenna
24- Transceiver
25- Decoder
26- wick
27- Tank mouth
28-Air flow control device
29- Touch display
30- Means of detection

### DESCRIPTION OF A REALIZATION PREFERRED

The present device for disinfection and purification of air and surfaces comprises a body (1) with an air inlet (3) and a hydroxyl radical output (4), which has inside it a low ozone emission ionizer (2) with pulse width modulation (PWM) power regulation, a removable tank (5) for a monoterpene reagent and a central processing unit (CPU) (6) with Wi-Fi and Bluetooth capability, that receives information from means of detection (30) about environment conditions, specifically, an internal air sensor (7), an external air sensor (8), an ozone sensor (9) and a luminosity sensor (10).

Operationally, the device has a room air intake (3) with a carbon filter (11) capable of retaining suspended particles which communicates with an Air intake duct (12) in which the internal air sensor (7) is located.

This Air intake duct (12) is connected to the removable tank (5) of monoterpene reagent which, together with the air, is fed into a reactive chamber (13) fitted with spiral guides (14), into which an ozone supply channel (15) flows, conveying the ozone produced from the ionizer (2) to the reactive chamber (13).

In the reactive chamber (13), thanks to the spiral guides (14), the filtered air coming from the Air intake duct (12), the monoterpene vapour generated by the means to cause the emanation of the reagent and the ozone generated in the ionizer (2) are mixed in an optimized way. The flow rate through the Air intake duct (12) and the ozone supply channel (15) is regulated by means of the suction of a turbine (16) with pulse width regulation (PWM), located at the output of the reactive chamber (13), to facilitate the chemical reaction generating the radical hydroxyl and adjusting to the volume required to cover the room.

Another novel aspect of the invention concerns the incorporation of means with safety protection that ensure proper use of the reagent, the use of an original product (understood as reagent authorized by the manufacturer) and the initial volume, and the remaining use of the monoterpene reagent tank. These means are constituted by a system to identifying the tank by radio frequency (RFID) (17) comprising a transponder (18) incorporated in the removable tank (5) and an RFID reader (22) integrated in the body (1) of the device. The transponder (18) comprises a chip (21), a radio transducer (20) and an antenna (19), while the RFID reader (22) comprises a transceiver (24), a decoder (25) and an antenna (23).

In the radio frequency identification (RFID) system (17), the RFID reader (22) integrated in the body (1) of the device, receives the RFID signal from the transponder (18) incorporated in the removable container (5), with the identification information of the tank content previously recorded in the chip (21) and sends it to the CPU (6).

The means for causing the monoterpene reagent to emanate consists of a wick (26) inserted in the removable tank (5) of reagent, which can be of various sizes and protrude a certain height from the mouth of the tank (27) of reagent which communicates with the Air intake duct (12). The monoterpene projects into through an air flow control device (28), which in the example shown is a changeable fixed part of various sizes, although it could be an electromechanical mobile system that throttles the flow section at that point, so that the monoterpene evaporates by vapour pressure and Venturi effect and is sucked through the Air intake duct (12) and introduced into the reagent cavity (13).

The central processing unit (6) is linked to a touch screen display (29) for device management.

## Claims

1. Device for disinfecting and purifying air and surfaces **characterized in that** it has a body (1) comprising:
- A removable tank (5) of monoterpene reagent incorporating:
• A wick (26) covered by an air flow control device (28) for adjustment of the amount of monoterpene vapour generated
• A transponder (18) that emits an RFID signal containing data related to the contained reagent which is picked up by an RFID reader (22) integrated into the body (1), **characterised in that** the device furthermore comprises:
- A reactive chamber (13) fitted with spiral-shaped guides (14) where the hydroxyl radicals are generated by mixing monoterpene vapour, ozone and air, into which converges an ozone supply channel (15) and an Air intake duct (12) equipped with a wick section (26) introduced inside it, and an internal air sensor (7) that measures the amount of volatile organic compounds (VOC), CO2 as well as relative humidity of the air passing through the air inlet duct (12) from the room via the air inlet (3).
- A central processing unit (CPU) (6) with WIFI and/or Bluetooth capability, which determines the level of pollution in the room from data received from the internal air sensor (7), from an ozone sensor (9) located at the hydroxyl radical output (4) and via WIFI or Bluetooth, from an external air sensor (8) which measures the amount of volatile organic compounds (VOC) outside the body (1) and depending on the level of pollution detected by the sensors (7) and (8), increases or decreases the rate of generation of hydroxyl radicals by modifying, by pulse width modulation (PWM) or by voltage, the power of a low ozone emission ionizer (2), the air flow control device (28) and the speed of a turbine (16) located at the output of the reactive chamber (13) whose suction level determines the supply of air and monoterpene vapour to the reactive chamber (13).
- A luminosity sensor (10) connected to the CPU (6) for automated selection between a high efficiency cycle where the CPU (6) operates the equipment at higher performance when light is detected and a low efficiency cycle where, in the absence of light, the CPU (6) reduces the activity of the elements that can produce the most noise, limits hydroxyl emission, and turns off all light and sound indicators.

2. Device for disinfecting and purifying air and surfaces according to claim 1, **characterised in that** the flow control device (28) consists of a changeable fixed part of various sizes that regulates the air flow in this area.

3. Device for disinfecting and purifying air and surfaces according to the first claim, **characterised in that** the passage control device (28) consists of an electromechanical moving element that throttles the passage section at that point.

4. Device for disinfecting and purifying air and surfaces according to the first claim, **characterised in that** the central processing unit (6) is connected to a touch screen LCD display (29) for managing the device, through which the user can observe and select different operating options and enter values.

## Patentansprüche

1. **Vorrichtung zur Desinfektion und Reinigung von Luft und Oberflächen, dadurch gekennzeichnet, dass** sie einen Körper (1) umfasst, bestehend aus:
∘ Einem **abnehmbaren Behälter** (5) für das Monoterpen-Reagens, der Folgendes enthält:
▪ Einen **Docht** (26), der von einer **Luftstromregelvorrichtung** (28) abgedeckt wird, um die Menge des erzeugten Monoterpendampfs zu regulieren
▪ Einen **Transponder** (18), der ein RFID-Signal sendet, das Daten über das enthaltene Reagens enthält und von einem im Körper (1) integrierten RFID-Lesegerät (22) empfangen wird, **dadurch gekennzeichnet, dass** die Vorrichtung ferner umfasst:
∘ Eine **Reaktionskammer** (13), ausgestattet mit spiralförmigen Führungen (14), in der Hydroxylradikale durch das Mischen von Monoterpendampf, Ozon und Luft erzeugt werden. In diese Kammer münden ein **Ozonzufuhrkanal** (15) und ein **Lufteinlasskanal** (12), der mit einem darin eingeführten **Dochtabschnitt** (26) versehen ist. Ein **interner Luftsensor** (7) misst die Menge an flüchtigen organischen Verbindungen (VOC), CO₂ sowie die relative Luftfeuchtigkeit der durch den Lufteinlasskanal (12) aus dem Raum über den **Lufteinlass** (3) strömenden Luft.
∘ Eine **Zentraleinheit (CPU)** (6) mit WLAN- und/oder Bluetooth-Funktion, die den Verschmutzungsgrad im Raum anhand der vom internen Luftsensor (7) empfangenen Daten, einem **Ozonsensor** (9), der sich am Ausgang der Hydroxylradikale (4) befindet, sowie über WLAN oder Bluetooth von einem externen Luftsensor (8), der die Menge an flüchtigen organischen Verbindungen (VOC) außerhalb des Körpers (1) misst, ermittelt. Abhängig von dem durch die Sensoren (7) und (8) festgestellten Verschmutzungsgrad erhöht oder verringert die CPU die Erzeugungsrate der Hydroxylradikale, indem sie mittels Pulsweitenmodulation (PWM) oder Spannungsregelung die Leistung eines **Ionisators mit geringer Ozonemission** (2), die Luftstromregelvorrichtung (28) und die Geschwindigkeit einer **Turbine** (16) anpasst, die sich am Ausgang der Reaktionskammer (13) befindet und deren Saugstärke die Zufuhr von Luft und Monoterpendampf zur Reaktionskammer (13) bestimmt.
o Einen **Lichtsensor** (10), der mit der CPU (6) verbunden ist, um automatisch zwischen einem **Hocheffizienzzyklus,** bei dem die CPU (6) das Gerät bei erkannter Lichtquelle mit höherer Leistung betreibt, und einem **Niedrigeffizienzzyklus,** bei dem die CPU (6) bei fehlendem Licht die Aktivität der Komponenten mit höherer Geräuschentwicklung reduziert, die Hydroxylabgabe einschränkt und alle Licht- und Tonanzeigen ausschaltet, zu wählen.

2. **Vorrichtung zur Desinfektion und Reinigung von Luft und Oberflächen** gemäß Anspruch 1, **dadurch gekennzeichnet, dass** die Luftstromregelvorrichtung (28) aus einem austauschbaren festen Teil verschiedener Größen besteht, das den Luftstrom in diesem Bereich reguliert.

3. **Vorrichtung zur Desinfektion und Reinigung von Luft und Oberflächen** gemäß Anspruch 1, **dadurch gekennzeichnet, dass** die Luftstromregelvorrichtung (28) aus einem elektromechanischen beweglichen Element besteht, das den Durchlassquerschnitt an dieser Stelle drosselt.

4. **Vorrichtung zur Desinfektion und Reinigung von Luft und Oberflächen** gemäß Anspruch 1, **dadurch gekennzeichnet, dass** die Zentraleinheit (CPU) (6) mit einem **LCD-Touchscreen** (29) verbunden ist, über den der Benutzer verschiedene Betriebsoptionen beobachten, auswählen und Werte eingeben kann.

## Revendications

1. Dispositif pour la désinfection et la purification d'air et de surfaces, **caractérisé en ce qu'**il comprend un corps (1) comprenant :
∘ Un réservoir amovible (5) de réactif monoterpénique, intégrant : • Une mèche (26) recouverte par un dispositif de contrôle du flux d'air (28) permettant de régler la quantité de vapeur de monoterpène générée
• Un transpondeur (18) qui émet un signal RFID contenant des données relatives au réactif contenu, lequel est capté par un lecteur RFID (22) intégré dans le corps (1), le dispositif étant **caractérisé en ce qu'**il comprend en outre :
∘ Une chambre réactive (13) équipée de guides en spirale (14) où les radicaux hydroxyles sont générés par le mélange de vapeur de monoterpène, d'ozone et d'air, dans laquelle convergent un canal d'alimentation en ozone (15) et un conduit d'admission d'air (12) équipé d'une section de mèche (26) introduite à l'intérieur, ainsi qu'un capteur d'air interne (7) mesurant la quantité de composés organiques volatils (COV), de CO2, ainsi que l'humidité relative de l'air traversant le conduit d'admission d'air (12) en provenance de la pièce via l'entrée d'air (3).
∘ Une unité centrale de traitement (CPU) (6) avec connectivité WIFI et/ou Bluetooth, qui détermine le niveau de pollution dans la pièce à partir des données reçues du capteur d'air interne (7), d'un capteur d'ozone (9) situé à la sortie des radicaux hydroxyles (4) et, via WIFI ou Bluetooth, d'un capteur d'air externe (8) qui mesure la quantité de composés organiques volatils (COV) à l'extérieur du corps (1). En fonction du niveau de pollution détecté par les capteurs (7) et (8), le CPU augmente ou diminue le taux de génération de radicaux hydroxyles en modifiant, par modulation de largeur d'impulsion (PWM) ou par tension, la puissance d'un ioniseur à faible émission d'ozone (2), le dispositif de contrôle du flux d'air (28) et la vitesse d'une turbine (16) située à la sortie de la chambre réactive (13), dont le niveau d'aspiration détermine l'apport en air et vapeur de monoterpène dans la chambre réactive (13).
∘ Un capteur de luminosité (10) connecté au CPU (6) permettant une sélection automatique entre un cycle haute efficacité, où le CPU (6) fait fonctionner l'équipement à des performances élevées lorsqu'il détecte de la lumière, et un cycle basse efficacité où, en l'absence de lumière, le CPU (6) réduit l'activité des éléments pouvant produire le plus de bruit, limite l'émission de radicaux hydroxyles, et éteint tous les indicateurs lumineux et sonores.

2. Dispositif pour la désinfection et la purification d'air et de surfaces selon la première revendication, **caractérisé en ce que** le dispositif de contrôle de flux (28) se compose d'une pièce fixe interchangeable de différentes tailles régulant le flux d'air dans cette zone.

3. Dispositif pour la désinfection et la purification d'air et de surfaces selon la première revendication, **caractérisé en ce que** le dispositif de contrôle de flux (28) consiste en un élément électromécanique mobile qui régule la section de passage à ce point.

4. Dispositif pour la désinfection et la purification d'air et de surfaces selon la première revendication, **caractérisé en ce que** l'unité centrale de traitement (6) est reliée à un écran tactile LCD (29) permettant la gestion du dispositif, grâce auquel l'utilisateur peut observer et sélectionner différentes options de fonctionnement et entrer des valeurs.
